(19) **Europäisches Patentamt European Patent Office Office européen des brevets**

(11) **EP 3 378 324 B1**

(12) **EUROPEAN PATENT SPECIFICATION**

(45) Date of publication and mention of the grant of the patent:
**10.05.2023 Bulletin 2023/19**

(21) Application number: **18170348.9**

(22) Date of filing: **24.05.2012**

(51) International Patent Classification (IPC):
**A23D 9/013** (2006.01)　　**A23L 33/115** (2016.01)
**A23L 33/10** (2016.01)　　**A23D 7/005** (2006.01)
**A23D 7/04** (2006.01)

(52) Cooperative Patent Classification (CPC):
**A23D 7/0053; A23D 7/04; A23L 29/10; A23L 33/15; A61P 3/02**

(54) **METHOD FOR PRODUCING OIL-IN-WATER EMULSIFIED FOOD PRODUCT COMPOSITION**

VERFAHREN ZUR HERSTELLUNG EINER NAHRUNGSMITTELPRODUKTZUSAMMENSETZUNG IN FORM EINER ÖL-IN-WASSER-EMULSION

PROCÉDÉ POUR LA FABRICATION D'UNE COMPOSITION DE PRODUIT ALIMENTAIRE ÉMULSIFIÉ HUILE-DANS-EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.05.2011 JP 2011118415**

(43) Date of publication of application:
**26.09.2018 Bulletin 2018/39**

(62) Document number(s) of the earlier application(s) in accordance with Art. 76 EPC:
**12789312.1 / 2 716 164**

(73) Proprietor: **Kaneka Corporation**
**Kita-ku,**
**Osaka 530-8288 (JP)**

(72) Inventors:
• **Kawashima, Yui**
**Hyogo, 676-8688 (JP)**
• **Inoue, Hiroaki**
**Hyogo, 676-8688 (JP)**
• **Yokota, Shinichi**
**Hyogo, 676-8688 (JP)**
• **Hamada, Kazuya**
**Hyogo, 676-8688 (JP)**
• **Sato, Masao**
**Hyogo, 676-8688 (JP)**

(74) Representative: **Hoffmann Eitle**
**Patent- und Rechtsanwälte PartmbB**
**Arabellastraße 30**
**81925 München (DE)**

(56) References cited:
EP-A1- 2 457 447　　JP-A- S6 025 934
US-A- 3 528 823　　US-A- 4 524 086
US-A1- 2008 131 576　　US-B1- 6 475 539

**Description**

Technical Field

The present invention relates to a method for producing an oil-in-water emulsified food product composition that can suppress negative effects caused by foaming during mixing without using an antifoaming agent or by using a small amount of an antifoaming agent and relates to an oil-in-water emulsified food product which is obtained by the method of the invention.

Background Art

In recent years, many nutritious food products to achieve an appropriate nutritional balance have been used not only as a portable food and a diet food that can be readily taken instead of a meal but also as a food product to be taken by people who are difficult to take nourishment by mouth due to advanced age, illness, wounds, or disabilities. Among them, many fluid diets have been developed to be used for tube feeding that is drawing attention in a medical field. For example, Patent Document 1 discloses an acidic type liquid enteral nutrient containing proteins, lipids, carbohydrates, vitamins, minerals, and emulsifiers.

However, the feeding of a fluid diet by the tube feeding may cause aspiration pneumonia due to the backflow of the fed fluid diet from the stomach into the gullet or may cause diarrhea due to the direct flow of the fed fluid diet to the small intestine without staying in the stomach. These problems are likely to be caused by feeding a fluid diet in a liquid shape having a low viscosity as described in Patent Document 1, for example. In order to solve such a problem, various semi-solidified nutrients have been developed. The semi-solidified nutrient is produced by mixing a thickening dietary fiber such as agar and pectin to preliminarily semi-solidify the nutrient (Patent Documents 2 to 4).

However, such a semi-solidified nutrient that has been semi-solidified as above has high viscosity and thus may be frequently difficult to be fed through a tube having a small diameter. To address this problem, there is a demand for a novel fluid diet that has a lower viscosity during feeding than that of a semi-solidified nutrient and thus is easily fed but is semi-solidified in the stomach to substantially the same degree as the semi-solidified nutrient and thus prevents gastroesophageal reflux and diarrhea.

Nutritious food products such as a fluid diet, a diet food, and a health drink, which are required to achieve an appropriate nutritional balance, are typically prepared by appropriately mixing proteins, oils and fats, minerals, water, emulsifiers, and other various components and emulsifying the mixture with, for example, a high-speed mixer in order to uniformly disperse these components (Patent Documents 1 to 5). However, the actual production and commercialization of a nutritious food product containing a large amount of thickening dietary fibers is generally believed to be difficult from the viewpoint of the yield during mixing and emulsion stability.

Patent Document 6 describes a foamable oil-in-water emulsified oil composition for chilled-distribution whipped creams.

Patent Document 7 describes a nutritional enteral formula having a pH of from 3.0 to 4.6 that contains high levels of macronutrients, vitamins and minerals. The enteral formula uses a stabilizing system comprising high methoxy pectin to stabilize the protein and a specific process to produce the formula.

Patent Document 8 describes a whippable food product reportedly having improved stability characteristics without detectable change in the organoleptic characteristics. The product comprises triglyceride fat, one or more sugars and an emulsifier component comprising destabilizing and stabilizing emulsifiers in amounts sufficient to stabilize the product.

Patent Document 9 describes an edible lauric hard butter composition suitable for the preparation of confectionery coatings. It uses a specific emulsifier ester blend that reportedly improves the performance of lauric hard butters in confectionery coatings.

Patent Document 10 describes a fluid shortening for use in baking that comprises an ester of polyglycerol, a normally solid triglyceride, an ester of propylene glycol and a normally liquid triglyceride.

Patent Document 11 describes a nutrient composition for preoperative or postoperative administration through the tracts that comprises proteins, saccharides, lipids, vitamins and minerals as main components and adding a specific amount of a specific oil or fat as the lipid.

Patent Document 12 describes an emulsified food composition that can be administered through a tube and that is semi-solidified inside the stomach to prevent gastro-esophageal reflux which promotes a feeling of fullness. The emulsified food composition is obtained by mixing at least a protein, a lipid, a carbohydrate and a thickening material and consequently has flowability until it goes into the stomach and is semi-solidified in the stomach.

Citation List

Patent Literatures

Patent Document 1: JP-A No. 2010-83774
Patent Document 2: Japanese Patent No. 4047363
Patent Document 3: International Publication WO 2007/026474
Patent Document 4: JP-A No. 2009-11229
Patent Document 5: JP-A No. 2009-106187
Patent Document 6: EP 2 457 447 A1
Patent Document 7: US 6 475 539 B1
Patent Document 8: US 2008/131576 A1
Patent Document 9: US 4 524 086 A
Patent Document 10: US 3 528 823 A
Patent Document 11: JP 60025934
Patent Document 12: JP 2011 147444

Summary of Invention

Technical Problem

In order to develop a food product to achieve an appropriate nutritional balance, the inventors of the present invention have studied a method for producing an oil-in-water emulsified food product composition containing proteins, oils and fats, emulsifiers, and other components, and have found the following problems: for example, the composition is vigorously foamed during mixing to largely lower the yield; the vigorous foaming causes poor emulsification in an emulsification process after the mixing and generates phase separation or aggregates after heat sterilization; and the oil-in-water emulsified food product composition has a higher air content, which deteriorates the storage stability of the food product composition if it contains components susceptible to oxidation, such as vitamins. It has been found that thickening dietary fibers, which are used to specifically develop a fluid diet product that has a low viscosity during feeding to be readily fed and is semi-solidified in the stomach, cause more vigorous foaming during mixing and thus are likely to cause the problems as above.

To address the problems, the inventors of the present invention have studied the method for adding commonly adopted antifoaming agents and foam suppressors as the means to suppress such foaming, but have found that any method fails to achieve a sufficient effect. For example, an emulsifier such as monoglyceride oleate and sorbitan trioleate was used as the antifoaming agent but achieved insufficient antifoaming effect. A silicone antifoaming agent such as a silicone emulsion that is commonly used as the antifoaming agent has high antifoaming performance, but the amount of the silicone antifoaming agent is limited under the Food Sanitation Law. For such a reason, the silicone antifoaming agent used within a predetermined amount has achieved insufficient antifoaming performance. These silicone antifoaming agents are ensured to have a certain degree of safety. However, increasing health-consciousness or others requires an emulsified food composition containing no silicone antifoaming agent from a social point of view. Accordingly, there has been a demand for a novel antifoaming or foam suppression means that does no require such a silicone antifoaming agent.

In view of the above circumstances, the present invention has an object to provide a method for producing a high quality oil-in-water emulsified food product composition that can suppress foaming of the oil-in-water emulsified food product composition during mixing, can generate no phase separation or aggregates or extremely suppress the generation of phase separation or aggregates after heat sterilization, and can maintain the stability of components susceptible to oxidation, such as vitamins during preservation if such a component is contained.

Solution to Problem

As a result of further studies, the inventors of the present invention have found that the problem can be solved by, preparing an oil-in-water emulsified food product composition containing proteins, thickening dietary fibers, oils and fats, and emulsifiers, preliminarily adding a highly hydrophilic emulsifier, which is typically dissolved in water and then mixed with oil, to an oil and fat to prepare an oil and fat containing the highly hydrophilic emulsifier, and then adding and mixing the oil and fat or a mixture of the oil and fat and water to water or an aqueous solution of water-soluble minerals, and by adjusting the oil and fat to have a ratio of medium chain fatty acids of more than 0.1% by weight and less than 100% by weight with respect to the total fatty acids, and have accomplished the present invention. In other words, the scope of the present invention is as below:

(1) A method for producing an oil-in-water emulsified food product composition includes adding an oil and fat containing a highly hydrophilic emulsifier or a mixture of an oil and fat containing a highly hydrophilic emulsifier and water to water or an aqueous solution of water-soluble minerals and mixing them, then adding a protein, a thickening dietary fiber, and optionally an additional component, and heating and sterilizing the composition; wherein the oil and fat contains a medium chain fatty acid in a ratio of more than 0.1% by weight and less than 100% by weight with respect to the total fatty acid composition; the highly hydrophilic emulsifier has an HLB value of 6.0 or more; the thickening dietary fiber is selected from gellan gum, carrageenan, pectin, agar, xanthan gum, locust bean gum, alginic acid, alginates, gum arabic, collagen, gelatin, curdlan, and poly-gamma-glutamic acid; and the pH of the oil-in-water emulsified food product composition, immediately after production, is more than 5.5.

(2) In the method for producing an oil-in-water emulsified food product composition according to the aspect (1), the highly hydrophilic emulsifier has an HLB value of 8.0 or more.

(3) In the method for producing an oil-in-water emulsified food product composition according to the aspect (1) or (2), the highly hydrophilic emulsifier has an HLB value of 11.0 or more.

(4) In the method for producing an oil-in-water emulsified food product composition according to any one of the aspects (1) to (3), the oil and fat contains the medium chain fatty acid in a ratio of 7.5% by weight or more and 74.9% by weight or less with respect to the total fatty acid composition.

(5) In the method for producing an oil-in-water emulsified food product composition according to any one of the aspects (1) to (4), the mixture of an oil and fat containing a highly hydrophilic emulsifier and water is a preliminarily emulsified mixture.

(6) The method for producing an oil-in-water emulsified food product composition according to any one of the aspects (1) to (5) wherein the method includes adding the thickening dietary fiber and then adding the protein.

(7) The method for producing an oil-in-water emulsified food product composition according to any one of the aspects (1) to (5) wherein the method includes adding the protein and then adding the thickening dietary fiber.

(8) The method for producing an oil-in-water emulsified food product composition according to any one of the aspects (1) to (5) wherein the method includes adding the protein and the thickening dietary fiber at the same time.

(9) In the method for producing an oil-in-water emulsified food product composition according to any one of the aspects (1) to (8), the oil-in-water emulsified food product composition has a viscosity of 25 cP or more and less than 1,000 cP after the heating and sterilizing the composition.

(10) In the method for producing an oil-in-water emulsified food product composition according to any one of the aspects (1) to (9), the thickening dietary fiber is contained in the oil-in-water emulsified food product composition in an amount of 0.05% w/v or more.

(11) In the method for producing an oil-in-water emulsified food product composition according to any one of the aspects (1) to (10), the thickening dietary fiber is alginic acid and/or an alginate.

(12) In the method for producing an oil-in-water emulsified food product composition according to any one of the aspects (1) to (11), the protein is contained in the oil-in-water emulsified food product composition in an amount of 1.0% w/v or more in terms of solid content.

(13) An oil-in-water emulsified food product composition is obtained by the method for producing an oil-in-water emulsified food product composition according to any one of the aspects (1) to (12), wherein the total concentration of emulsifiers in the oil-in-water emulsified food product composition is 5.0% by weight or more with respect to an oil and fat.

Advantageous Effects of Invention

A method for producing an oil-in-water emulsified food product composition of the present invention can suppress foaming of an oil-in-water emulsified food product composition during mixing without using a silicone or other antifoaming agents, a foam suppressor, or other agents or by using a small amount of such an agent to thus improve the yield, can stabilizes the emulsion after heat sterilization to generate no aggregates or phase separation or to extremely suppress the generation of aggregates or phase separation, and can consequently provide a high quality oil-in-water emulsified food product composition. The method can provide a higher quality oil-in-water emulsified food product composition capable of maintaining the stability of components susceptible to oxidation, such as vitamins during preservation, if such a component is contained.

An oil-in-water emulsified food product composition containing thickening dietary fibers is more vigorously foamed during preparation. Thus, the present invention is particularly preferred to produce an oil-in-water emulsified food product composition that contains the thickening dietary fiber, and has flowability in a neutral region, and is semi-solidified in an acidic region.

Description of Embodiments

The present invention, which is defined by the claims, will be described in detail hereinafter.

A method for producing an oil-in-water emulsified food product composition of the present invention is characterized by including adding an oil and fat containing a highly hydrophilic emulsifier or a mixture of an oil and fat containing a highly hydrophilic emulsifier and water to water or an aqueous solution of water-soluble minerals (hereinafter also abbreviated as "water or the like") and mixing them, then adding a protein, a thickening dietary fiber as described herein, and optionally an additional component, and heating and sterilizing the composition. The oil and fat contains a medium chain fatty acid in a ratio of more than 0.1% by weight and less than 100% by weight; the highly hydrophilic emulsifier has an HLB value of 6.0 or more; the thickening dietary fiber is selected from gellan gum, carrageenan, pectin, agar, xanthan gum, locust bean gum, alginic acid, alginates, gum arabic, collagen, gelatin, curdlan, and poly-gamma-glutamic acid; and the pH of the oil-in-water emulsified food product composition, immediately after production, is more than 5.5.

Typically, a highly hydrophilic emulsifier selected as an emulsifier is preliminarily mixed with water or an aqueous solution and then is mixed with predetermined components. However, in the present invention, as described above, the highly hydrophilic emulsifier is mixed with an oil and fat to give an oil and fat containing the highly hydrophilic emulsifier, and then the oil and fat is directly added to and mixed with water or an aqueous solution of water-soluble minerals, or a mixture of the oil and fat and water is added to and mixed with water or an aqueous solution of water-soluble minerals in advance. After the process, a protein and a dietary fiber is added. Such a method can markedly suppress the foam generated during mixing each component. It is supposed that the suppression of the foaming during mixing as above suppresses insufficient emulsification due to the foam in an emulsification treatment process after the mixing, and this stabilizes the emulsion after a heat sterilization process subsequent to the emulsification treatment to generate no aggregates or phase separation or to extremely suppress the generation of aggregates or phase separation. In addition, as a result of the reduction in air content in the finally obtained oil-in-water emulsified food product composition, it is supposed that the oil-in-water emulsified food product composition can maintain the stability of components susceptible to oxidation, such as vitamins during preservation, if such a component is contained.

In the present invention, "during mixing" means during mixing all components of an oil-in-water emulsified food product composition and does not include emulsification treatment after the mixing.

The present invention employs the oil and fat containing a highly hydrophilic emulsifier as described above. The method of preparing the oil and fat is not specifically limited as long as the method is as above. For example, a highly hydrophilic emulsifier may be suspended in an oil and fat and the mixture may be stirred with a common stirrer or other means to prepare a suspension.

The present invention may also employ a mixture that is obtained by preliminarily mixing the oil and fat containing a highly hydrophilic emulsifier obtained as above and water. Examples of the method of preparing such a mixture include, but are not necessarily limited to, a method of suspending a highly hydrophilic emulsifier in an oil and fat, stirring the mixture with a common stirrer or other means as described above to yield a suspension, mixing the suspension with a predetermined amount of water, and stirring the mixture with a common stirrer or other means and a method of preliminarily emulsifying the mixture with an emulsification apparatus in place of the stirrer or after stirring with a stirrer. The preliminary emulsification here means emulsification of a composition containing a highly hydrophilic emulsifier, an oil and fat, and a predetermined amount of water alone before the composition is added to water or an aqueous solution of water-soluble minerals. In the present invention, in order to further suppress the foaming during mixing and to further stabilize the emulsion after heat sterilization, a preliminarily emulsified mixture of an oil and fat containing a highly hydrophilic emulsifier and water is preferably used. It is supposed that such a preemulsion that is obtained by preliminary emulsification of the mixture of an oil and fat containing a highly hydrophilic emulsifier and an appropriate amount of water is used to stabilize the preliminary dispersed state of the oil and fat in water with a highly hydrophilic emulsifier, and this can further effectively suppress the foaming.

For the preliminary emulsification, the mixing ratio of the oil and fat containing a highly hydrophilic emulsifier to water is not particularly limited, but water is preferably used in an amount of 100 to 600 parts by weight with respect to 100 parts by weight of the total amount of an oil and fat and a highly hydrophilic emulsifier. In order to further suppress the foaming, as for the ratio of water to be mixed with the oil and fat containing a highly hydrophilic emulsifier, water is preferably mixed in an amount of 300 parts by weight or more and more preferably 600 parts by weight with respect to 100 parts by weight of the oil and fat. The emulsification apparatus used for the preliminary emulsification is not particularly limited, and non-limiting examples include low-speed stirrers such as a propeller mixer and a turbine mixer, high-speed rotary homogenizers such as a homomixer, and high-pressure/high-speed jet homogenizers such as a grinder (colloid mill) and a high-pressure jet homogenizer. In order to further increase the affinity between a highly hydrophilic emulsifier and an oil and fat and to suppress the foaming during mixing, a high-pressure homogenizer is preferably used. The preliminary emulsification is preferably carried out at a working pressure of 5 MPa or more, more preferably 10 MPa or more, even more preferably 20 MPa or more, and particularly preferably 30 MPa or more.

The oil-in-water emulsified food product composition of the present invention contains, as essential components, a protein, an oil and fat, a highly hydrophilic emulsifier, and water, and further contains a thickening dietary fiber as described later. For such a composition, the order of addition of each component is not particularly limited as long as an oil and fat containing a highly hydrophilic emulsifier or a mixture of an oil and fat containing a highly hydrophilic emulsifier and water (hereinafter also called "oil and fat containing a highly hydrophilic emulsifier or the like") is first added to water or an aqueous solution of water-soluble minerals as described above. After the process of adding an oil and fat containing a highly hydrophilic emulsifier or the like to water or the like and mixing them, a protein may be added and then a thickening dietary fiber may be added; a thickening dietary fiber may be added and then a protein may be added; or both may be added at the same time. In addition, after the process of adding the predetermined oil and fat or the like to water or the like and mixing them, stirring and mixing may be carried out after each component is added in a respective process, may be carried out after all components are added, or may be carried out in other arbitrary manners.

In the present invention, other components may be added in addition to the protein, the oil and fat, the highly hydrophilic emulsifier, the water-soluble mineral, the thickening dietary fiber, and water, as described later. These additional components may be added in a similar order to that for the protein and the thickening dietary fiber.

In the method for producing an oil-in-water emulsified food product composition of the present invention, in order to suppress the phase separation or the aggregates after heat sterilization, it is preferable that all components of the oil-in-water emulsified food product composition are mixed and then the mixture containing all the components is emulsified immediately before the heat sterilization.

The emulsification treatment can be carried out with various emulsification apparatuses described above. In order to stabilize the emulsion after the heat sterilization, a high-pressure homogenizer is preferably used. The emulsification is preferably carried out at a working pressure of 20 MPa or more, more preferably 30 MPa or more, even more preferably 35 MPa or more, furthermore preferably 40 MPa or more, and particularly preferably 45 MPa or more.

In order to further stabilize the emulsified state of the oil-in-water emulsified food product composition after the heat sterilization process and to suppress the generation of aggregates in the oil-in-water emulsified food product composition, emulsification is preferably carried out twice or more. For twice emulsification with a high-pressure homogenizer, the first emulsification is preferably carried out at 5 MPa or more, more preferably at 10 MPa or more, furthermore preferably at 15 MPa or more, and particularly preferably at 20 MPa or more, and the second emulsification is preferably carried out at 10 MPa or more, more preferably at 20 MPa or more, even more preferably at 30 MPa or more, furthermore preferably at 40 MPa or more, and particularly preferably at 50 MPa or more.

For twice or more emulsification with a high-pressure homogenizer, the treatment is preferably performed while the working pressure increases step-by-step.

In the present invention, after the mixing of each component of the oil-in-water emulsified food product composition and the emulsification, (i) the emulsion may be directly subjected to heat sterilization treatment and then filled into a desired container, or (ii) the emulsion may be filled into a desired container and then subjected to heat sterilization treatment. Such a heat sterilization treatment can prevent the emulsified food composition from suffering physical property change due to, for example, microorganisms. The heat sterilization treatment may be any method suitable for (i) or (ii) and may be any of pasteurization, high-temperature sterilization, and ultrahigh-temperature short-time sterilization as long as the emulsified food composition can be prevented from suffering physical property change due to, for example, microorganisms. The sterilization method may be any of UHT sterilization, HTST sterilization, retort sterilization, autoclave sterilization, plate sterilization, and tubular sterilization. However, the oil-in-water emulsified food product composition is likely to generate aggregates after the heat sterilization by retort sterilization. Thus, the production process of the present invention achieves a higher effect when the oil-in-water emulsified food product composition is sterilized by retort sterilization.

The material and the shape of the container for filling is not particularly limited and a soft bag, a pouch such as an aluminum pouch, a paper pack, a can, a bottle, and other containers may be used. The container preferably has such a shape as to prevent the contamination of microorganisms or others that change physical properties of an emulsified food composition. In order to suppress the reduction in nutritional components such as vitamins, the container is preferably made of a material having light blocking properties and/or gas barrier properties, but a transparent container can be used. All components including a protein, an oil and fat, an emulsifier, and other components added as necessary in the oil-in-water emulsified food product composition that is obtained by the production method of the present invention can be filled in the same container.

The oil-in-water emulsified food product composition and each component will next be described.

The type of the protein usable in the present invention is not particularly limited and any of animal proteins and plant proteins may be used. The protein in the present invention includes degradation products such as hydrolysates of proteins. Examples of the animal protein include proteins derived from milk (for example, casein and whey protein), proteins derived from eggs, and hydrolysates of them. Examples of the plant protein include proteins derived from soybeans, proteins derived from wheat, proteins derived from green peas, proteins derived from rice, and hydrolysates of them. These proteins may be used singly or in combination of a plurality of them. In order to suppress the foaming during mixing and to suppress the aggregates and the phase separation after heat sterilization, the protein type is preferably plant proteins. Among them, plant proteins derived from legume seeds are more preferred and plant proteins derived from soybeans and/or hydrolysates of them are even more preferred. A nitrogen source may be not only proteins but also peptides and amino acids.

The amount of proteins is not particularly limited. In order to suppress the foaming during mixing, the oil-in-water emulsified food product composition preferably contains a protein in an amount of 8.0% w/v or less, more preferably 6.0% w/v or less, even more preferably 5.0% w/v or less, furthermore preferably 4.5% w/v or less, and particularly preferably 4.0% w/v or less. As for the lower limit of the protein concentration, the oil-in-water emulsified food product composition preferably contains a protein in an amount of 0.1% w/v or more, more preferably 0.5% w/v or more, even more preferably 1.0% w/v or more, furthermore preferably 2.0% w/v or more, and particularly preferably 3.0% w/v or more.

The type of the oil and fat usable in the present invention is not particularly limited as long as medium chain fatty acids are contained in a ratio of more than 0.1% by weight and less than 100% by weight with respect to the total fatty acid composition, and various oils and fats commonly usable in a food field may be used. Examples of such an oil and fat include oils and fats derived from plants, including soybean oil, corn oil, palm oil, palm kernel oil, safflower oil, coconut oil, and rapeseed oil and oils and fats derived from animals, including fish oil, beef tallow, lard, and cream. In the present invention, a fatty acid means an aliphatic monocarboxylic acid obtained by hydrolysis of an oil and fat, and a medium chain fatty acid means a fatty acid having 8 to 12 carbon atoms.

Arbitrary oils and fats derived from plants and animals can be used without treatment as long as an oil and fat contains a triglyceride having at least one acyl group with a carbon number of 8 or more and 12 or less as above. In addition, an oil and fat mixture of a purified medium chain fatty acid triglyceride (MCT) having an acyl group with a carbon number of 8 or more and 12 or less with an oil and fat containing, as an acyl group, a fatty acid except particular medium chain fatty acids, such as a short chain fatty acid triglyceride (SCT) with a carbon number of 6 or less, a saturated fatty acid including palmitic acid ($C_{16}$), a monounsaturated fatty acid including oleic acid ($C_{18}$), an n-3 polyunsaturated fatty acid including docosahexaenoic acid ($C_{22}$), eicosapentaenoic acid ($C_{20}$), and $\alpha$-linolenic acid ($C_{18}$), and an n-6 polyunsaturated fatty acid including linoleic acid ($C_{18}$), may be used, and a mixture of an oil and fat derived from a natural product with a purified medium chain fatty acid triglyceride may also be used. Usable oils and fats are not limited to them. Several types of oils and fats may be combined so as to give the fatty acid composition ratio described in "Dietary Reference Intakes for Japanese (2010)" (May 29, 2009, the Ministry of Health, Labor and Welfare) and such a mixture may be added. A medium chain fatty acid triglyceride is also abbreviated as "MCT" below. MCT is the abbreviation of medeium chain triglyceride.

In order to further suppress the foaming during mixing and to further suppress the generation of aggregates after heat sterilization or to extremely reduce the generation, the lower limit of the amount of medium chain fatty acids in an oil and fat to be used is preferably 5% by weight or more, more preferably more than 7% by weight, even more preferably 7.5% by weight or more, furthermore preferably 8% by weight or more, particularly preferably 10% by weight or more, and most preferably 13% by weight or more, with respect to the total fatty acid composition. The upper limit is preferably 80% by weight or less, more preferably 74.9% by weight or less, even more preferably 50% by weight or less, and furthermore preferably 40% by weight or less.

The type of the oil and fat usable in the present invention is not particularly limited as long as the ratio of saturated fatty acids is more than 8.7% by weight and less than 100% by weight with respect to the total fatty acid composition, and various oils and fats commonly usable in a food field can be used. Here, the saturated fatty acid is an aliphatic monocarboxylic acid obtained by hydrolysis of an oil and fat and is a fatty acid having no unsaturated bond on its carbon chain. The oil and fat used in the present invention may be any oil and fat containing a triglyceride having at least one acyl group with no unsaturated bond. In order to further suppress the foaming during mixing and to further suppress the generation of aggregates after heat sterilization or to extremely reduce the generation, the lower limit of the ratio of saturated fatty acids in an oil and fat to be used is preferably 18.7% by weight or more and more preferably 32.9% by weight or more with respect to the total fatty acid composition. The upper limit is preferably 80.7% by weight or less.

In the present invention, the concentration of the oil and fat in the oil-in-water emulsified food product composition is not particularly limited. In order to suppress the foaming during mixing, the concentration is preferably 0.5% w/v or more, more preferably 1.0% w/v or more, even more preferably 1.5% w/v or more, furthermore preferably 2.0% w/v or more, particularly preferably 2.5% w/v or more, and most preferably 3.0% w/v or more. The upper limit is preferably 40% w/v or less, more preferably 30% w/v or less, even more preferably 20% w/v or less, and particularly preferably 10% w/v or less.

The present invention can employ any emulsifier having high hydrophilicity and the type is not particularly limited. Using a highly hydrophilic emulsifier to prepare the composition by the production process as above can suppress the foaming during mixing. Usable examples of the emulsifier include lecithin, lysolecithin, sucrose fatty acid esters, glycerin fatty acid esters, organic acid monoglycerides, polyglycerol fatty acid esters, polyglycerol condensed ricinoleic acid esters, sorbitan fatty acid esters, and propylene glycol fatty acid esters. These emulsifiers may be used singly or in combination of two or more of them. In order to further suppress the foaming during mixing, the emulsifier to be used preferably has an HLB value of 8.0 or more, more preferably has an HLB value of 9.0 or more, even more preferably has an HLB value of more than 9.5, furthermore preferably has an HLB value of 10.0 or more, and particularly preferably has an HLB value of 11.0 or more.

In the present invention, "highly hydrophilic" means having an HLB value of 6.0 or more.

Among the highly hydrophilic emulsifiers, in order to stabilize the emulsion after heat sterilization, at least one emulsifier selected from lysolecithin, organic acid monoglycerides, and polyglycerol fatty acid esters is preferably used, at least one emulsifier selected from lysolecithin and polyglycerol fatty acid esters is more preferably used, and lysolecithin is even more preferably used. Among lysolecithins, a lysolecithin derived from soybeans is preferably used. For polyglycerol fatty acid esters, decaglycerol monostearate is more preferably used.

In the present invention, a highly hydrophilic emulsifier may be used in combination with additional emulsifiers (for example, a highly lipophilic emulsifier). In this case, the highly hydrophilic emulsifier is preferably contained in a ratio of 50% by weight or more, more preferably 60% by weight or more, even more preferably 70% by weight or more, furthermore preferably 80% by weight or more, and particularly preferably 90% by weight or more, in all emulsifiers.

The total concentration of emulsifiers in the oil-in-water emulsified food product composition is appropriately set within a range not impairing the advantageous effects of the present invention. However, in order to stabilize the emulsion after heat sterilization, the concentration is preferably 5.0% by weight or more, more preferably 7.0% by weight or more, even more preferably 8.0% by weight or more, and particularly preferably 10.0% by weight or more with respect to an oil and fat.

The water used as the component in the oil-in-water emulsified food product composition is preferably purified waters such as distilled water and ion-exchanged water.

Subject to the appended claims, the present invention can employ any type of dietary fibers. Here, the dietary fiber means indigestible polysaccharides that are carbohydrates not digested by human digestive enzymes.

Among the dietary fibers, in order to semi-solidify the composition in the stomach, dietary fibers that are added to increase the viscosity of the whole composition are added.

Adding a thickening dietary fiber may cause more vigorous foaming during mixing, but the production method of the present invention can be adopted to markedly suppress the foaming during mixing. On this account, the production method of the present invention is particularly effective on an oil-in-water emulsified food product composition containing a thickening dietary fiber as a component.

As described above, subject to the appended claims, the present invention may employ any type of the thickening dietary fiber. In order to provide an oil-in-water emulsified food product composition having flowability in a region except an acidic region, such as a neutral region and being semi-solidified in an acidic region, the thickening dietary fiber to be used preferably semi-solidifies the composition in an acidic region. According to the present invention, the thickening dietary fiber is selected from gellan gum, carrageenan, pectin, agar, xanthan gum, locust bean gum, alginic acid, alginates, gum arabic, collagen, gelatin, curdlan, and poly-gamma-glutamic acid. These thickening dietary fibers may be used singly or in combination of a plurality of them. In order to sufficiently semi-solidify an oil-in-water emulsified food product composition in an acidic region, a thickening dietary fiber preferably gelates in an acidic region, and examples include gellan gum, carrageenan, pectin, curdlan, alginic acid, and alginates. Among them, in order to provide an emulsified food composition keeping flowability in a neutral region and being sufficiently semi-solidified in an acidic region, at least one selected from gellan gum, carrageenan, alginic acid, alginates, and pectin is more preferred, at least one selected from alginic acid, alginates, and pectin is even more preferred, and alginic acid and alginates are particularly preferred.

The gellan gum is produced by fermentation of Pseudomonas elodea and is straight chain polysaccharides composed of a repeating unit of four sugars, glucose, glucuronic acid, glucose, and rhamnose. Among gellan gums, in order to effectively semi-solidify the food composition in an acidic region, deacylated gellan gum is preferred.

The carrageenan is extracted from red algae and is composed of salts of polysaccharide sulfates including galactose. Among carrageenans, in order to effectively semi-solidify the food composition in an acidic region, kappa-carrageenan is preferred.

The pectin is a high methoxyl pectin having an esterification degree of 50% or more, a low methoxyl pectin having an esterification degree of less than 50%, or a mixture of them. Examples of the pectin include pectin derived from citrus fruits such as lemon, lime, orange, and grapefruit and apple pectin. The type of the pectin is not particularly limited. In order to effectively semi-solidify the food composition in an acidic region, a low methoxyl pectin is preferred.

The alginic acid and the alginates are hydrophilic colloidal polysaccharides obtained by extraction from seaweeds. Among the alginic acid and the alginates, in order to suppress the flowability to a low viscosity in a neutral region and to suppress the foaming during mixing, a 1% by weight aqueous solution (20°C) of alginic acid or an alginate preferably has a viscosity of 900 cP or less, more preferably 600 cP or less, even more preferably 400 cP or less, furthermore preferably 200 cP or less, and particularly preferably 100 cP or less.

The agar is not particularly limited, is commonly obtained by extraction from red algae, and is polysaccharides containing agarose and agaropectin. The type of the agar is not particularly limited, and any of agars having various physical properties such as high jelly strength, low jelly strength, easy solubility, high viscosity, and low viscosity may be used.

The xanthan gum is polysaccharides obtained by the accumulation and fermentation of glucose and others by Xanthomonas compestris.

The locust bean gum is a natural water-soluble gum derived from seeds of locust tree, a Leguminosae plant.

The gum arabic is polysaccharides obtained by drying a resin derived from an acacia, a Leguminosae plant.

The collagen is a fibrous protein constituting dermal tissue and other tissues of mammals and includes a hydrolysis product.

The gelatin is produced by degradation and purification of collagen.

The poly-gamma-glutamic acid is produced by a Bacillus edible microorganism, Bacillus subtilis chungkookjang or other microorganisms and is an anionic polymer harmless and edible.

The curdlan is polysaccharides mainly containing $\beta$-1,3-glucose linkages and is exemplified by polysaccharides produced by Alcaligenes or Agrobacterium bacteria.

In the present invention, the appropriate concentration of a thickening dietary fiber in the oil-in-water emulsified food product composition varies depending on the type of the thickening dietary fiber and is preferably about 0.05% w/v or more and 5% w/v or less in terms of solid content in the oil-in-water emulsified food product composition. A thickening dietary fiber in an amount of less than 0.05% w/v achieves insufficient semi-solidification in an acidic region and this may achieve the insufficient prevention effect on gastroesophageal reflux. A thickening dietary fiber in an amount of more than 5% w/v may cause vigorous foaming during mixing. The concentration of a thickening dietary fiber is in terms of solid content hereinbelow unless otherwise specified.

For alginic acid and/or alginates used as the thickening dietary fiber, the lower limit concentration (total concentration when two compounds are used) is preferably 0.05% w/v or more, more preferably 0.1% w/v or more, even more preferably 0.2% w/v or more, furthermore preferably 0.5% w/v or more, and particularly preferably 1.0% w/v or more. The upper limit is preferably 10% w/v or less, more preferably 5.0% w/v or less, even more preferably 3.0% w/v or less, and particularly preferably 2.0% w/v or less.

In the present invention, in addition to the protein, the oil and fat, the thickening dietary fiber, and the emulsifier, the composition may further include other nutritional components (for example, vitamins, minerals (water-soluble and water-insoluble minerals), trace elements, and carbohydrates), functional components (for example, coenzyme Q10), fruit juice, flavors, antifoaming agents, and other components.

In the present invention, the oil-in-water emulsified food product composition optionally contains water-soluble minerals as its component. The water-soluble minerals are not particularly limited, and examples include salts of alkali metals, such as sodium salts and potassium salts of alkali metals. When a water-soluble mineral is added, the order of addition is not particularly limited. An aqueous mineral solution may be added either before oil is added or in a mixing step after oil is added.

The type of the water-insoluble minerals is not particularly limited. In order to maintain the flowability in a neutral region and to suppress the foaming during mixing, salts containing a divalent or higher valent metal ion are preferably a compound water-insoluble in a neutral region. Alternatively, salts containing a divalent or higher valent metal ion are preferably coated with a composition that is water-insoluble in a neutral region and is water-soluble in an acidic region. Among the divalent or higher valent metal ions, salts containing a calcium ion or a magnesium ion are more preferably a compound water-insoluble in a neutral region, and examples of such a compound include calcium citrate, calcium silicate, calcium stearate, calcium carbonate, tricalcium phosphate, calcium monohydrogen phosphate, magnesium oxide, magnesium stearate, magnesium carbonate, trimagnesium phosphate, magnesium hydroxide, and magnesium silicate. Examples of the composition for coating salts containing a divalent or higher valent metal ion include, but are not necessarily limited to, gelatin, agar, and curdlan.

The concentration of the minerals (including water-soluble minerals and water-insoluble minerals) in the oil-in-water emulsified food product composition is appropriately set within a range not impairing the advantageous effects of the present invention in consideration of the nutritional balance and other factors.

A non-thickening dietary fiber is an optional component and the type is not particularly limited if it is added. Examples of the non-thickening dietary fiber optionally added include water-insoluble dietary fibers such as cellulose, wheat bran, soybean dietary fiber, and corn fiber and water-soluble dietary fibers such as indigestible dextrin, polydextrose, and degradation products of guar gum.

The concentration of the non-thickening dietary fiber in the oil-in-water emulsified food product composition is appropriately set within a range not impairing the advantageous effects of the present invention in consideration of the nutritional balance and other factors. It is feared that the addition of an excess amount of a non-thickening dietary fiber may cause vigorous foaming during mixing. Thus, in order to suppress the foaming during mixing, the concentration of a non-thickening dietary fiber is preferably 5.0% w/v or less, more preferably 4.0% w/v or less, even more preferably 3.0% w/v or less, furthermore preferably 2.0% w/v or less, and particularly preferably 1.0% w/v or less.

The present invention can employ any type of carbohydrates. The carbohydrate is an optional component. In order to further semi-solidify the composition in an acidic region, a carbohydrate is preferably added. Here, the carbohydrate is a carbohydrate that is not contained in dietary fibers.

Examples of the carbohydrate used in the present invention include, but are not necessarily limited to, monosaccharides such as glucose, disaccharides such as sucrose, oligosaccharides such as fructooligosaccharide, and polysaccharides such as dextrin. These carbohydrates may be used singly or in combination of a plurality of them. The concentration of the carbohydrates in the oil-in-water emulsified food product composition is appropriately set within a range not impairing the function of the present invention in consideration of the nutritional balance and other factors.

The viscosity of the emulsified food composition of the present invention during mixing is not particularly limited, but a larger viscosity during mixing is likely to cause more vigorous foaming during mixing. However, the production method of the present invention can effectively suppress the foaming and thus the viscosity may be comparatively high during mixing. In order to further suppress the foaming during mixing, the viscosity during mixing is preferably less than 1,000 cP, more preferably less than 500 cP, even more preferably less than 300 cP, and particularly preferably less than 250 cP.

A lower viscosity is likely to reduce the foaming during mixing and thus the lower limit of the viscosity during mixing is not set. A viscosity of about 25 cP or more during mixing is likely to increase the foam during mixing if the method of the present invention is not applied. Thus, the present invention can markedly achieve the foaming suppressive effect on a composition having a viscosity of about 25 cP or more during mixing. However, the degree of foaming may vary depending on components or other factors, and a causal relationship is not necessarily clear, but the present invention may markedly achieve the foaming suppressive effect on a composition having a viscosity of 50 cP or more, 75 cP or more, or 100 cP or more.

Here, cP (centipoise) is the unit representing viscosity and 1 cP is 1 mPa·s.

The oil-in-water emulsified food product composition obtained by the production method of the present invention may have any degree of flowability as long as the oil-in-water emulsified food product composition has flowability except in an acidic region (for example, in a neutral region) after heat sterilization. However, an oil-in-water emulsified food product composition used as a tube-feeding enteral nutrient preferably has an upper limit viscosity of less than 1,000 cP, more preferably 500 cP or less, even more preferably 300 cP or less, furthermore preferably 250 cP or less, after heat sterilization. The lower limit viscosity is preferably 25 cP or more, more preferably 50 cP or more, even more preferably 70 cP or more, and particularly preferably 80 cP or more.

The oil-in-water emulsified food product composition that is obtained by the production method of the present invention and that contains, for example, a thickening dietary fiber may be semi-solidified in an acidic region, but the viscosity of the semi-solidified composition is not particularly limited as long as a feeling of fullness is accelerated and gastroesophageal reflux is prevented. In order to effectively prevent gastroesophageal reflux or diarrhea, the semi-solidified composition preferably has a viscosity of 1,000 cP or more, more preferably 2,000 cP or more, even more preferably 5,000 cP or more, furthermore preferably 8,000 cp or more, and particularly preferably 10,000 cP or more.

Here, whether an oil-in-water emulsified food product composition is semi-solidified or not in the stomach is basically judged by visual examination on properties of the mixture of artificial gastric juice and the composition. As described above, "being semi-solidified" in the present invention is having a lower flowability or having no flowability. Specifically, a semi-solidified substance is, for example, a viscous fluid such as yoghurt and a substance maintaining a certain shape, such as pudding and chawan-mushi (steamed egg custard). Accordingly, whether the mixture of artificial gastric juice and an oil-in-water emulsified food product composition is, for example, a viscous fluid such as yoghurt or a substance maintaining a certain shape, such as pudding and chawan-mushi or not is visually examined.

The oil-in-water emulsified food product composition obtained by the production method of the present invention may have any pH. The pH varies depending on the type of components contained in the oil-in-water emulsified food product composition. According to the present invention and in order to maintain the viscosity low in a neutral region and to suppress the foaming during mixing, the pH immediately after production is more than 5.5, more preferably 5.7 or more, even more preferably 6.0 or more, and particularly preferably 6.5 or more. The lower limit pH of the neutral region in the present invention is more than 5.5, more preferably 5.7 or more, even more preferably 6.0 or more, and particularly preferably 6.5 or more. The upper limit pH is preferably 10.0 or less, more preferably 9.0 or less, even more preferably 8.5 or less, and particularly preferably 8.0 or less. In the present invention, "immediately after production" is immediately after heat sterilization of an oil-in-water emulsified food product composition as above and also includes a period until the composition is supplied to various applications described later.

The oil-in-water emulsified food product composition obtained by the production method of the present invention may have any energy density. In order to suppress the foaming during mixing, the upper limit is preferably 3.0 kcal/ml or less, more preferably 2.5 kcal/ml or less, even more preferably 2.0 kcal/ml or less, and particularly preferably 1.5 kcal/ml or less. The lower limit is preferably 0.1 kcal/ml or more, more preferably 0.3 kcal/ml or more, and even more preferably 0.5 kcal/ml or more.

The oil-in-water emulsified food product composition obtained by the production method of the present invention is a high quality oil-in-water emulsified food product composition in which the emulsion can be stabilized after heat sterilization to generate no aggregates or phase separation or to extremely suppress the generation.

"Aggregates" mean a substance that can be formed during the production or preservation of an oil-in-water emulsified food product composition to cause tube clogging during tube feeding or to cause a rough texture during oral nutrition of the oil-in-water emulsified food product composition. The aggregates are supposed to be formed by single and/or complex participation of, for example, particles, particle aggregates, water-soluble dietary fibers, metallic compounds, proteins, emulsifiers, oils and fats, and other nutritional components (such as carbohydrates and dietary fibers) in an oil-in-water emulsified food product composition.

The amount of formed aggregates that may cause tube clogging, rough texture, or other troubles can be evaluated by the weight of filtration substances of an oil-in-water emulsified food product composition through, for example, nylon mesh or filter paper. The measurement method will be specifically described in Examples later.

In the oil-in-water emulsified food product composition of the present invention, particles contained in an oil-in-water emulsified food product composition in a neutral region may have any particle size (median size). In order to effectively suppress the generation of aggregates, the particle size (median size) of the particles is preferably 5.0 $\mu$m or less, more preferably 4.5 $\mu$m or less, even more preferably 4.0 $\mu$m or less, and particularly preferably 3.5 $\mu$m or less.

Here, "particles" in the present invention mean a substance dispersed and/or suspended in a liquid as a continuous phase in an oil-in-water emulsified food product composition. The "particles" may be composed of any substance that is dispersed and/or suspended in a liquid and is supposed to be formed by single and/or complex participation of, for example, proteins, oils and fats, carbohydrates, thickening dietary fibers, and emulsifiers capable of being contained in the composition. The particle size (median size) of particles can be calculated based on the particle size distribution determined with, for example, a particle size distribution analyzer employing laser diffraction/scattering method.

The measurement method of the particle size (median size) of particles will be described below with reference to an example using a laser diffraction/scattering particle size distribution analyzer (manufactured by Horiba, Ltd., LA-950) as a particle size distribution analyzer.

The particle size is analyzed with the laser diffraction/scattering particle size distribution analyzer under the following conditions: the dispersion medium is distilled water; the sample refractive index is 1.600-0.000i; the dispersion medium refractive index is 1.333; the circulation rate is 13; the stirring speed is 2; and the sample concentration is adjusted so that the light transmission factor (R) is 90 to 80% and the transmission factor (B) is 90 to 70% during analysis.

For the analysis of particle size distribution under the condition above, the particle size distribution of particles is shown by a distribution curve where the horizontal axis is particle size ($\mu$m) and the vertical axis is frequency (%) based on volume in the analysis by the laser diffraction/scattering method. From the particle size distribution analyzed in this manner, a median size (D50) and an average diameter (arithmetic mean value of frequency distribution) are calculated, and these values are regarded as the particle size of particles in the present invention. The particle size can be analyzed by both methods in which a sample is sonicated and is not sonicated.

Utility forms of the oil-in-water emulsified food product composition obtained by the production method of the present invention will be described below.

The oil-in-water emulsified food product composition can be fed by conventional methods such as oral nutrition and tube feeding. For example, the liquid food product composition can be directly fed through the mouth or can be fed dropwise through a tube from a container hanged on a stand. The liquid food product composition can also be forcibly fed, for example, by using a pump or a pressure bag or by pressurizing a container by hand, but the feeding method is not limited to them.

The oil-in-water emulsified food product composition has flowability in a neutral region, contains a few aggregates, and is a smooth liquid, thus providing good swallowing feeling. A common solid food has dry-and-crumbly texture when it is fed and thus is indicated to have disadvantages, for example, it is difficult to be swallowed, has insufficient texture, and is likely to require water during feeding. However, the emulsified food composition of the present invention enables easy oral nutrition. The emulsified food composition, which has good swallowing feeling, can be used as a fluid diet, a nursing diet, and a medical diet for patients suffering from difficulty in swallowing or chewing.

Among the oil-in-water emulsified food product compositions, an oil-in-water emulsified food product composition that is semi-solidified in an acidic region (that is, in the stomach) can provide a feeling of satiety that is difficult to be provided by conventional gel-like portable foods or gel-like diet foods and should promote a feeling of satiety as compared with common foods. Such a food product composition can be accordingly used for diet foods, for example.

The food product composition, which has flowability in a neutral region and is semi-solidified in an acidic region, is easily fed and should have the effect of preventing gastroesophageal reflux. The food product composition is thus used for, in addition to oral nutrition, a fluid diet, a nursing diet, and a medical diet that are also fed through a nasogastric tube or a gastrostomy catheter. In particular, the food product composition, which is readily fed and effectively prevents gastroesophageal reflux, is suitable as a fluid diet, specifically, as an enteral nutrition food product or an enteral nutrient. For example, as the fluid diet, the food product composition, which has flowability and contains no aggregates or markedly suppresses the generation of aggregates in a neutral region, that is, until the composition enters the stomach, eliminates pressurizing that is required when a fluid diet is semi-solidified with, for example, agar or a thickening agent and is fed, and saves time and labor of nurses, caregivers, and other people. The food product composition, which contains no aggregates or markedly suppresses the generation of aggregates, can easily pass through a tube to the stomach without clogging. This enables easy tube feeding through, for example, a nasogastric tube or a gastrostomy catheter. The food product composition is unlikely to pollute the inside of a tube, and this simplifies the treatment of the tube after feeding. The food product composition is effectively semi-solidified in an acidic region, that is, in the stomach to prevent gastro-esophageal reflux, thus achieves the gastroesophageal reflux prevention effect in a similar manner to the case in which a food for preventing nausea is fed in addition to a fluid diet. The food product composition can also reduce the worsening of bedsore of people to be fed due to a seated position for a long time, while saving time, labor, and cost. The fed food product composition is discharged from the stomach at a slower speed than a common liquid fluid diet that is not semi-solidified in the stomach, and thus can prevent diarrhea.

An additive for an oil-in-water emulsified food product composition, which is not part of the present invention, will next be described.

The additive for an oil-in-water emulsified food product composition is an additive for an oil-in-water emulsified food product composition that includes an oil and fat containing a medium chain fatty acid in a ratio of more than 0.1% by weight and less than 100% by weight with respect to the total fatty acid composition and a highly hydrophilic emulsifier. The medium chain fatty acid is preferably contained in an amount of 6% by weight or more and 70% by weight or less with respect to the additive for an oil-in-water emulsified food product composition, and the ratio of (the amount of the emulsifier in terms of % by weight with respect to the additive for an oil-in-water emulsified food product composition)/(the amount of the medium chain fatty acid in terms of % by weight with respect to the additive for an oil-in-water emulsified food product composition) is preferably 0.13 or more and 1.5 or less. The additive for an oil-in-water emulsified food product composition is added as a part of a composition and differs from the composition.

Using the additive for an oil-in-water emulsified food product composition during a production process of an oil-in-water emulsified food product composition can suppress foaming of the oil-in-water emulsified food product composition during mixing and can suppress the generation of aggregates and phase separation in the oil-in-water emulsified food product composition after heating even without using a silicone or other antifoaming agents, a foam suppressor, or other agents or by using a small amount of such an agent.

The additive for an oil-in-water emulsified food product composition preferably contains the medium chain fatty acid in an amount of 6% by weight or more and 70% by weight or less, more preferably 6.8% by weight or more and 68.1% by weight or less, and even more preferably 13.5% by weight or more and 68.1% by weight or less. An additive containing the medium chain fatty acid in an amount of less than 6% by weight fails to suppress the foaming during mixing and is likely to reduce the yield. An additive containing the medium chain fatty acid in an amount of more than 70% by weight is likely to generate aggregates after heat sterilization.

The amount (% by weight) of the medium chain fatty acid is determined as follows: the quantitative determination below of a medium chain fatty acid determines the weight of the medium chain fatty acid contained in an additive for an oil-in-water emulsified food product composition; and then the ratio of the weight of the medium chain fatty acid with respect to the total weight of the additive for an oil-in-water emulsified food product composition can be calculated as the amount (% by weight) of the medium chain fatty acid.

The ratio of (the amount of the emulsifier in terms of % by weight with respect to the additive for an oil-in-water emulsified food product composition)/(the amount of the medium chain fatty acid in terms of % by weight with respect to the additive for an oil-in-water emulsified food product composition) is preferably 0.13 or more and 1.5 or less, more preferably 0.13 or more and 1.33 or less, and even more preferably 0.19 or more and 1.33 or less. An additive having a ratio of less than 0.13 is likely to generate aggregates after heat sterilization. An additive having a ratio of more than 1.5 is unlikely to suppress the foaming during mixing to reduce the yield.

In the additive for an oil-in-water emulsified food product composition, the ratio of saturated fatty acids with respect to the total fatty acid composition in an oil and fat is not particularly limited as long as the ratio is more than 8.7% by weight and less than 100% by weight. In order to further suppress the foaming during mixing and to further suppress the generation of aggregates after heat sterilization or to extremely reduce the generation, the lower limit of the ratio of saturated fatty acids in an oil and fat to be used in the additive is preferably 18.7% by weight or more and more preferably 32.9% by weight or more with respect to the total fatty acid composition. The upper limit is preferably 80.7% by weight or less.

The additive for an oil-in-water emulsified food product composition may further include, in addition to the oil and fat and the highly hydrophilic emulsifier described above, other components (for example, minerals (including water-soluble and water-insoluble minerals) and vitamins), carotenoids, coenzyme Q10, lutein, fruit juice, flavors, and others, and these components can be designed depending on each purpose. In order to further suppress the foaming, the additive for an oil-in-water emulsified food product composition is preferably composed of the oil and fat and the highly hydrophilic emulsifier above alone.

The additive for an oil-in-water emulsified food product composition may be mixed with other nutritional components (for example, minerals (including water-soluble and water-insoluble minerals) and vitamins), carotenoids, coenzyme Q10, lutein, fruit juice, flavors, and others before the additive is mixed with water.

The additive for an oil-in-water emulsified food product composition can be used to produce an oil-in-water emulsified food product composition.

The production method will be briefly described with reference to an example of the additive for an oil-in-water emulsified food product composition including a predetermined oil and fat and a predetermined emulsifier. First, a predetermined oil and fat is mixed with a predetermined emulsifier to prepare an additive for an oil-in-water emulsified food product composition. Then, the additive is added to and mixed with water or an aqueous solution of water-soluble minerals. Then, a protein or a protein and an additional component are added, and the mixture is subjected to heat sterilization to produce an oil-in-water emulsified food product composition. Needless to say, a similar constitution to that previously described in the production method of the present invention can be specifically adopted.

The method for producing an oil-in-water emulsified food product composition by using the additive for an oil-in-water emulsified food product composition is not limited to the method above.

The present invention can provide an oil-in-water emulsified food product composition containing the additive for an oil-in-water emulsified food product composition, which is obtained by the method of the present invention

Examples

The present invention will be described in further detail with reference to examples below, but the present invention is not intended to be limited to the examples.

<Identification of Aggregates in Oil-in-Water Emulsified Food Product Composition>

The properties of an oil-in-water emulsified food product composition were visually examined and the amount of aggregates was calculated in accordance with the method below.

The tare weight of a nylon mesh (HC-58 (manufactured by NYTAL), mesh: 264 inches) was measured (regarded as [nylon mesh tare weight 2]). With a Buchner funnel (pore size: 2 mm) having a diameter of 11 cm, 300 ml of an oil-in-water emulsified food product composition was filtered through the nylon mesh under reduced pressure to collect aggregates. After filtration, the collected aggregates were dried together with the nylon mesh at 60°C for 20 minutes. After drying, the whole was cooled to room temperature and the dried aggregates were weighed together with the nylon mesh (regarded as [aggregate weight]). The amount of aggregates was calculated in accordance with the calculation formula below.

$$[\text{Amount of aggregates (g)}] = [\text{Aggregate weight}] - [\text{Nylon mesh tare weight 2}]$$

Evaluation standards are as follows:

* "Presence": aggregates are visually observed, 10 mg or more
* "Absence": no aggregates are visually observed, 10 mg or less

<Ratio of Foam during Mixing>

All components constituting an oil-in-water emulsified food product composition were mixed. The mixture was left for 10 minutes. The composition was transferred into a graduated cylinder before emulsification treatment and left for 5 minutes. The scales of the interface between foam and liquid level and the interface between the foam and air were read, and the ratio was calculated in accordance with the calculation formula below.

$$[\text{Ratio of foam (\%)}] = ([\text{Interface (ml) between foam and air}] - [\text{Interface (ml) between foam and liquid level}])/1{,}100\,\text{ml} \times 100$$

Evaluation standards are as follows:

"○": less than 12%, "×": 12% or more

< Measurement Method of Viscosity>

The viscosity of an oil-in-water emulsified food product composition was determined as follows:
The viscosity was determined with a Brookfield viscometer (manufactured by Tokimec, Inc.). Into a glass container having an inner diameter of 60 mm, 200 ml of an oil-in-water emulsified food product composition (25°C) was charged, and a No. 2 rotor was used at a rotation speed of 60 rpm. After one minute of the rotation, the measured value was read.

<Measurement Method of Semi-Solidification Degree>

Into a 50-ml screw cap tube, 20 g of an artificial gastric juice (pH 1.2, 2.0 g/L of sodium chloride, 7.0 ml/L of hydrochloric acid) at 37°C prepared in accordance with the disintegration test method in Japanese Pharmacopoeia was poured, and the poured artificial gastric juice was weighed (regarded as [artificial gastric juice amount]).
Next, into the tube, 10 g of an oil-in-water emulsified food product composition was charged, and the total weight ([total weight before suction filtration]) of the screw cap tube, the artificial gastric juice added, and the oil-in-water emulsified food product composition added was measured. After measurement, the tube was fixed to a fixture in a chamber of "HL-2000 HybriLinker (manufactured by UVP Laboratory Products)" and was gently stirred for 10 minutes under a condition of a temperature of 37°C and a speed of "MIN." After stirring, the mixture was filtered under reduced pressure through a nylon mesh (0.420 mm = 40 mesh; manufactured by Sogo Laboratory Glass Works Co., Ltd.). The tare weight [nylon mesh tare weight] of the nylon mesh was previously measured. Then, the nylon mesh together with the residue was disposed on paper towel and left for 2 minutes to remove excess water, and the nylon mesh was weighed (regarded as [semi-solidified substance weight after filtration]). The plastic tube alone after the content liquid was discharged was weighed (regarded as [tube weight after filtration]), and the semi-solidification degree was calculated in accordance with the calculation formula (Formula (1)) below.
For the evaluation standard, a sample having a semi-solidification degree of 40% or more is regarded as good.
[Mathematical Formula 1]

$$\mathrm{Semi\text{-}solidification\ degree}\,(\%) = \frac{[\text{Semi-solidified substance weight after filtration}] - [\text{Nylon mesh tare weight}]}{[\text{Total weight before suction filtration}] - [\text{Artificial gastric juice amount}] - [\text{Tube weight after filtration}]} \times 100 \quad \ldots \mathrm{Formula}(1)$$

Quantitative Determination Method of Medium Chain Fatty Acid and Saturated Fatty Acid>

(Pretreatment)

In an about 20-ml test tube with a cap, a drop of a sample was charged and 0.5 ml of isooctane was further added to dissolve the sample. Next, 0.5 ml of sodium methylate was added, and the tube was sealed with the cap and was left in a constant-temperature water bath at 85°C for 15 minutes. After cooling, 2 ml of a saturated aqueous sodium chloride solution and 2 ml of isooctane were added, then the whole was vigorously shaken, and the isooctane layer was analyzed by gas chromatography. The analysis condition was as follows:

(Analysis Condition for Gas Chromatography)

* Gas chromatograph: Agilent 6890N (manufactured by Agilent Technologies, Inc.)
* Column: HR-SS-10, 0.25 μm × 0.25 mm ID × 50 m (manufactured by Shinwa Chemical Industries Ltd.)
* Injector: sprit/sprit injector, sprit mode measurement, 265°C
* Detector: FID 265°C
* Column temperature: 180°C, isothermal × 60 minutes
* Carrier gas: He, 1 cm/min

(Production Example 1) Method for Adding Oil and Fat and Emulsifier

<Preparation of Oil-in-Water Emulsified Food Product Composition>

An oil-in-water emulsified food product composition was prepared in accordance with the formulation shown in Table 1.
Lysolecithin was added to a formulated oil while the mixture was stirred with a mechanical stirrer at a speed of 500 to 600 rpm, and a suspension (also serving as an additive) was prepared. Distilled water warmed at 60°C was measured and charged in a 2-L container, and the suspension (additive) was added. Then, in accordance with the order shown in Table 2, each component was added and mixed and consequently all the components were mixed. After mixing, the mixture was left for 10 minutes and then was transferred into a 2-L graduated cylinder. The mixture was further left for 5 minutes, and the ratio of foam was determined. After measurement, the mixture was emulsified with a Manton Gaulin high-pressure homogenizer (Rannie 2000: manufactured by APV) (the first step: 20 MPa, the second step: 50 MPa). After preparation, each soft bag was filled with 300 ml of the emulsified mixture and was subjected to retort sterilization (sterilization temperature: 123°C, sterilization time: 8 minutes 30 seconds).

As for the suspension (additive), the ratio of the amount (% by weight) of the emulsifier with respect to the suspension (additive) and the ratio of the amount (% by weight) of the medium chain fatty acids with respect to the suspension (additive) can be calculated by using the amounts of the formulated oil and lysolecithin in Table 1 and the ratio of the medium chain fatty acids. (The amount of the emulsifier in terms of % by weight with respect to the additive) = 0.34/(0.34 + 3.4) × 100 = 9.09% by weight, and (the amount of the medium chain fatty acid in terms of % by weight with respect to the additive) = 3.4 × 0.149/(0.34 + 3.4) × 100 = 13.5% by weight. Accordingly, for the suspension, namely, the additive for an oil-in-water emulsified food product composition, the ratio of (the amount of the emulsifier in terms of % by weight with respect to the additive for an oil-in-water emulsified food product composition)/(the amount of the medium chain fatty acid in terms of % by weight with respect to the additive for an oil-in-water emulsified food product composition) is about 0.67.

[Table 1]

| Component | Amount |
|---|---|
| Soybean protein [*1] | 4.4% w/v |
| Formulated oil [*2] | 3.4% w/v |
| Lysolecithin [*3] | 0.34% w/v |
| Carbohydrates | 12% w/v |
| Sodium alginate | 1.0% w/v |
| Minerals | 0.87% w/v |
| Vitamins | 0.05% w/v |
| Distilled water | Balance |
| Total amount | 1100 mL |
| *1: The amount of soybean protein mixed is based on solid content. *2: The ratio of rapeseed oil/corn oil/palm kernel oil is 1/1.2/1. (The ratio of medium chain fatty acids is 14.9% by weight, and the ratio of saturated fatty acids is 32.9%) *3: The HLB value is about 12. | |

[Table 2]

| Addition order | Production Example 1 |
|---|---|
| 1 | Formulated oil [*2]+Lysolecithin *4 |
| 2 | Sodium alginate |
| 3 | Soybean protein |
| 4 | Carbohydrates |
| 5 | Minerals |
| 6 | Vitamins |
| *4: The suspension in which lysolecithin was suspended in a formulated oil. | |

(Comparative Examples 1)

Oil-in-water emulsified food product compositions (Comparative Examples 1-1 and 1-2) were prepared in accordance with the formulations shown in Table 1.

Distilled water warmed at 60°C was measured and charged in a 2-L container. Then, in accordance with the order shown in Table 3, each component was added and mixed while the mixture was stirred with a mechanical stirrer at a speed of 500 to 600 rpm, and consequently all the components were mixed. After mixing, the mixture was left for 10 minutes and then was transferred into a 2-L graduated cylinder. The mixture was further left for 5 minutes, and the ratio of foam was determined. After measurement, the mixture was emulsified with a Manton Gaulin high-pressure homogenizer (Rannie 2000: manufactured by APV) (the first step: 20 MPa, the second step: 50 MPa). After preparation, each soft bag was filled with 300 ml of the emulsified mixture and was subjected to retort sterilization (sterilization temperature: 123°C, sterilization time: 8 minutes 30 seconds).

[Table 3]

| Addition order | Comparative Example 1-1 | Comparative Example 1-2 |
|---|---|---|
| 1 | Lysolecithin | Lysolecithin |
| 2 | Sodium alginate | Sodium alginate |
| 3 | Formulated oil *2 | Soybean protein |
| 4 | Soybean protein | Carbohydrates |
| 5 | Carbohydrates | Minerals |
| 6 | Minerals | Vitamins |
| 7 | Vitamins | Formulated oil |

<Evaluation Result of Production Example 1 and Comparative Examples 1>

The presence or absence of aggregates and the ratio of foam of the oil-in-water emulsified food product compositions of Production Example 1 and Comparative Examples 1 prepared as above were determined as above. Table 4 (Production Example 1) and Table 5 (Comparative Examples 1-1 and 1-2) show the evaluation results. As shown in Tables 4 and 5, the composition prepared by the method of suspending lysolecithin in a formulated oil and then mixing the suspension with water (Production Example 1) further suppressed the foaming during mixing and reduced the ratio of foam to less than 10% as compared with the compositions prepared by the method of separately mixing lysolecithin and a formulated oil (the method of separately mixing lysolecithin and a formulated oil with water) (Comparative Examples 1-1 and 1-2). Production Example 1 reduced the ratio of foam by about 5% as compared with Comparative Examples 1. However, this is supposed to achieve a large economic effect because several tons of the composition is produced in actual production.

The suppression of the foaming prevented the oil-in-water emulsified food product composition prepared by the method of Production Example 1 from causing phase separation after heat sterilization and reduced the amount of aggregates to 10 mg or less.

The composition of Production Example 1 had a viscosity of 109 cP and a semi-solidification degree of 49.6%. The result revealed that the oil-in-water emulsified food product composition prepared in Production Example 1 had flowability in a neutral region and was semi-solidified in an artificial gastric juice.

[Table 4]

| Evaluation item | | Production Example 1 |
|---|---|---|
| Amount of aggregates | [mg] | 7.4 |
| Presence or absence of aggregates (visual examination) | [-] | Absence |
| Ratio of foam | [%] | 8.6 |
| Viscosity (after sterilization) | [cP] | 109 |
| Semi-solidification degree | [%] | 49.6 |

[Table 5]

| Evaluation item | | Comparative Example 1-1 | Comparative Example 1-2 |
|---|---|---|---|
| Amount of aggregates | [mg] | 7.0 | 17.2 |
| Presence or absence of aggregates (visual examination) | [-] | Absence | Absence |
| Ratio of foam | [%] | 14.5 | 74.5 |

(Production Examples 2, Comparative Examples 2) Compounding Ratios of Medium Chain Fatty Acid and Saturated Fatty Acid

<Preparation of Oil-in-Water Emulsified Food Product Composition>

Oil-in-water emulsified food product compositions (Production Examples 2-1 to 2-4 and Comparative Examples 2-1 to 2-3) were prepared in accordance with the formulations shown in Table 6 (the formulation for the emulsified food composition) and Table 7 (the type of the oil and fat and the ratios of medium chain fatty acids and saturated fatty acids).

Distilled water warmed at 60°C was measured and charged in a 2-L container. Then, each component was added and mixed in accordance with the order shown in Table 8 while the mixture was stirred with a mechanical stirrer at a speed of 500 to 600 rpm (lysolecithin and an oil and fat were added as a suspension (also serving as an additive) in which lysolecithin was suspended in the oil and fat described in Table 7). After mixing, the mixture was left for 10 minutes and then was transferred into a 2-L graduated cylinder. The mixture was further left for 5 minutes, and the ratio of foam was determined. After measurement, the mixture was emulsified with a Manton Gaulin high-pressure homogenizer (Rannie 2000: manufactured by APV) (the first step: 20 MPa, the second step: 50 MPa). Then, each soft bag was filled with 300 ml of the emulsified mixture and was subjected to retort sterilization (sterilization temperature: 123°C, sterilization time: 8 minutes 30 seconds).

[Table 6]

| Component | Amount |
|---|---|
| Soybean protein *1 | 4.4% w/v |
| Oil and fat (* specifically described in Table 7) | 3.4% w/v |
| Lysolecithin | 0.34% w/v |
| Carbohydrates | 12% w/v |
| Sodium alginate | 1.0% w/v |
| Minerals | 0.87% w/v |
| Vitamins | 0.05% w/v |
| Distilled water | Balance |
| Total amount | 1100 mL |

[Table 7]

| No. | Type of oil and fat | Ratio of medium chain fatty acid *5 | Ratio of saturated fatty acid *6 |
|---|---|---|---|
| Comparative Example 2-1 | MCT purified oil | 100% by weight | 100% by weight |
| Production Example 2-1 | MCT purified oil/ rapeseed oil | 74.9% by weight | 77.3% by weight |
| Production Example 2-2 | Palm kernel oil | 53.1% by weight | 80.7% by weight |

(continued)

| No. | Type of oil and fat | Ratio of medium chain fatty acid *5 | Ratio of saturated fatty acid *6 |
|---|---|---|---|
| Production Example 2-3 | Formulated oil *2 | 14.9% by weight | 32.9% by weight |
| Production Example 2-4 | Palm kernel oil/rapeseed oil | 7.5% by weight | 18.7% by weight |
| Comparative Example 2-2 | Rapeseed oil | 0.1% by weight | 8.7% by weight |
| Comparative Example 2-3 | Corn oil | 0% by weight | 13% by weight |

*5 The ratio (% by weight) of medium chain fatty acids having 8 to 12 carbon atoms with respect to the total fatty acids.
*6: The ratio (% by weight) of saturated fatty acids with respect to the total fatty acids.

[Table 8]

| Addition order | Production Example 2, Comparative Example 2 |
|---|---|
| 1 | Oil and fat+Lysolecithin |
| 2 | Sodium alginate |
| 3 | Soybean protein |
| 4 | Carbohydrates |
| 5 | Minerals |
| 6 | Vitamins |

<Evaluation Result of Production Examples 2 and Comparative Examples 2>

The presence or absence of aggregates and the ratio of foam of the oil-in-water emulsified food product compositions (Production Examples 2-1 to 2-4 and Comparative Examples 2-1 to 2-3) prepared as above were determined as above. Table 9 and Table 10 show the evaluation results. As shown in Table 9 and Table 10, the compositions prepared by using the oil and fat having a medium chain fatty acid ratio of 7.5% by weight or more and a saturated fatty acid ratio of 18.7% by weight or more (Production Examples 2-1 to 2-4, Comparative Example 2-1) suppressed the foaming during mixing and reduced the ratio of foam to about 10% or less as compared with the compositions prepared by using the oil and fat having a medium chain fatty acid ratio of 0.1% by weight or less and a saturated fatty acid ratio of 8.7% by weight or less (Comparative Examples 2-2 to 2-3). In addition to the method of suspending a highly hydrophilic emulsifier in an oil and fat and then adding the suspension to water, increasing the ratio of medium chain fatty acids in an oil and fat to be used suppressed the foaming during mixing. However, in the composition prepared by using the oil and fat having a medium chain fatty acid ratio of 100% by weight and a saturated fatty acid ratio of 100% by weight, aggregates were observed after heat sterilization. It is supposed that an oil and fat having a medium chain fatty acid ratio of 100% by weight and a saturated fatty acid ratio of 100% by weight is used to destabilize an emulsified state. The result showed that the use of an oil and fat having a medium chain fatty acid ratio of more than 0.1% by weight and less than 100% by weight can further suppress the foaming during mixing, can reduce the amount of aggregates to 10 mg or less after heat sterilization, and can suppress the viscosity to 150 cP or less after heat sterilization.

The compositions of Production Examples 2 had a viscosity of about 100 to 200 cP and all the compositions had a semi-solidification degree of 40.0% or more. The result revealed that the compositions prepared in Production Examples 2 had flowability in a neutral region and were semi-solidified in an artificial gastric juice.

[Table 9]

| Evaluation item | | Comparative Example 2-1 | Production Example 2-1 | Production Example 2-2 | Production Example 2-3 |
| --- | --- | --- | --- | --- | --- |
| | | MCT purified oil (100% by weight [7]) | MCT purified oil /rapeseed oil (74.9% by weight [7]) | Palm kernel oil (53.1% by weight [7]) | Formulated oil (14.9 % byweight [7]) |
| Amount of aggregates | [mg] | 19.5 | 1.0 | 2.9 | 7.4 |
| Presence or absence of aggregates (visual examination) | [-] | Presence | Absence | Absence | Absence |
| Viscosity (after sterilization) | [cP] | 152.5 | 123.0 | 114.0 | 1000 |
| Ratio of foam | [%] | 5.5 | 5.0 | 5.0 | 8.6 |
| Semi-solidification degree | [%] | 41.1 | 41.7 | 47.5 | 49.6 |
| *7: Medium chain fatty acid amount | | | | | |

[Table 10]

| Evaluation item | | Production Example 2-4 | Comparative Example 2-2 | Comparative Example 2-3 |
| --- | --- | --- | --- | --- |
| | | Palm kernel oil /rapeseed oil (7.5% by weight [7]) | Rapeseed oil (0.1% by weight [7]) | Corn oil (0% by weight [7]) |
| Amount of aggregates | [mg] | 7.9 | 25.1 | 79.2 |
| Presence or absence of aggregates (visual examination) | [-] | Absence | Absence | Presence |
| Viscosity (after sterilization) | [cP] | 87.0 | 94.5 | 96.5 |
| Ratio of foam | [%] | 10.9 | 13.6 | 12.7 |
| Semi-solidification degree | [%] | 47.4 | 51.0 | 53.3 |

(Production Example 3) Preliminary Emulsification

<Preparation of Oil-in-Water Emulsified Food Product Composition>

An oil-in-water emulsified food product composition was prepared in accordance with the formulation shown in Table 1.

To about 600 parts by weight of distilled water in a 1-L container, 100 parts by weight of a suspension (also serving as an additive) in which lysolecithin was suspended in a formulated oil was added, and the whole was stirred with a mechanical stirrer. After stirring, the mixture was preliminarily emulsified with a Manton Gaulin high-pressure homogenizer (Rannie 2000: manufactured by APV) at a pressure of 20 MPa to afford a pre-emulsion.

Distilled water warmed at 60°C was measured and charged in a 2-L container, and the pre-emulsion was added and mixed while the mixture was stirred with a mechanical stirrer at a speed of 500 to 600 rpm. Then, each component was added and mixed in accordance with the order shown in Table 11 and consequently all the components were mixed. After mixing, the mixture was left for 10 minutes and then was transferred into a 2-L graduated cylinder. The mixture was further left for 5 minutes, and the ratio of foam was determined. After measurement, the mixture was emulsified with a Manton Gaulin high-pressure homogenizer (Rannie 2000: manufactured by APV) (the first step: 20 MPa, the second step: 50 MPa). After preparation, each soft bag was filled with 300 ml of the emulsified mixture and was subjected to retort sterilization (sterilization temperature: 123°C, sterilization time: 8 minutes 30 seconds).

[Table 11]

| Addition order | Production Example 3 |
|---|---|
| 1 | Formulated oil *2 +Lysolecithin+Water *8 |
| 2 | Sodium alginate |
| 3 | Soybean protein |
| 4 | Carbohydrates |
| 5 | Minerals |
| 6 | Vitamins |
| *8: Pre-emulsion | |

<Evaluation Result of Production Example 3>

The presence or absence of aggregates and the ratio of foam of the oil-in-water emulsified food product composition prepared as above were determined as above. Table 12 shows the evaluation result. As shown in Table 12, the composition prepared by suspending lysolecithin in a formulated oil to yield a suspension (also serving as an additive), then mixing the suspension with an appropriate amount of water, and preliminarily emulsifying the mixture (Production Example 3) suppressed the foaming during mixing and reduced the ratio of foam to less than 10% with respect to the total amount as compared with the composition prepared without preliminary emulsification (Production Example 1).

The suppression of the foaming prevented the composition prepared by the method of Production Example 3 from causing phase separation after heat sterilization and reduced the amount of aggregates to 10 mg or less.

The composition of Production Example 3 had a viscosity of 98 cP and a semi-solidification degree of 55.9%. The result revealed that the composition prepared in Production Example 3 had flowability in a neutral region and was semi-solidified in an artificial gastric juice.

[Table 12]

| Evaluation item | | Production Example 3 (Preliminarily emulsified) |
|---|---|---|
| Amount of aggregates | [mg] | 2.2 |
| Presence or absence of aggregates (visual examination) | [-] | Absence |
| Ratio of foam | [%] | 7.7 |
| Viscosity (after sterilization) | [cP] | 98 |
| Semi-solidification degree | [%] | 55.9 |

**Claims**

1. A method for producing an oil-in-water emulsified food product composition, the method comprising:

adding an oil and fat containing a highly hydrophilic emulsifier or a mixture of an oil and fat containing a highly hydrophilic emulsifier and water to water or an aqueous solution of water-soluble minerals and mixing them; then adding a protein, a thickening dietary fiber, and optionally an additional component; and
heating and sterilizing the composition;
wherein
the oil and fat containing a medium chain fatty acid in a ratio of more than 0.1% by weight and less than 100% by weight with respect to the total fatty acid composition;
the highly hydrophilic emulsifier has an HLB value of 6.0 or more;
the thickening dietary fiber is selected from gellan gum, carrageenan, pectin, agar, xanthan gum, locust bean gum, alginic acid, alginates, gum arabic, collagen, gelatin, curdlan, and poly-gamma-glutamic acid; and
the pH of the oil-in-water emulsified food product composition, immediately after production, is more than 5.5.

2. The method for producing an oil-in-water emulsified food product composition according to claim 1, wherein the highly hydrophilic emulsifier has an HLB value of 8.0 or more.

3. The method for producing an oil-in-water emulsified food product composition according to claim 1 or 2, wherein the highly hydrophilic emulsifier has an HLB value of 11.0 or more.

4. The method for producing an oil-in-water emulsified food product composition according to any one of claims 1 to 3, wherein the oil and fat contains the medium chain fatty acid in a ratio of 7.5% by weight or more and 74.9% by weight or less with respect to the total fatty acid composition.

5. The method for producing an oil-in-water emulsified food product composition according to any one of claims 1 to 4, wherein the mixture of an oil and fat containing a highly hydrophilic emulsifier and water is a preliminarily emulsified mixture.

6. The method for producing an oil-in-water emulsified food product composition according to any one of claims 1 to 5, the method comprising adding the thickening dietary fiber and then adding the protein.

7. The method for producing an oil-in-water emulsified food product composition according to any one of claims 1 to 5, the method comprising adding the protein and then adding the thickening dietary fiber.

8. The method for producing an oil-in-water emulsified food product composition according to any one of claims 1 to 5, the method comprising adding the protein and the thickening dietary fiber at the same time.

9. The method for producing an oil-in-water emulsified food product composition according to any one of claims 1 to 8, wherein the oil-in-water emulsified food product composition has a viscosity of 25 cP or more and less than 1,000 cP after the heating and sterilizing the composition.

10. The method for producing an oil-in-water emulsified food product composition according to any one of claims 1 or 9, wherein the thickening dietary fiber is contained in the oil-in-water emulsified food product composition in an amount of 0.05% w/v or more.

11. The method for producing an oil-in-water emulsified food product composition according to any one of claims 1 to 10, wherein the thickening dietary fiber is alginic acid and/or an alginate.

12. The method for producing an oil-in-water emulsified food product composition according to any one of claims 1 to 11, wherein the protein is contained in the oil-in-water emulsified food product composition in an amount of 1.0% w/v or more in terms of solid content.

13. An oil-in-water emulsified food product composition obtained by the method for producing an oil-in-water emulsified food product composition according to any one of claims 1 to 12, wherein the total concentration of emulsifiers in the oil-in-water emulsified food product composition is 5.0% by weight or more with respect to an oil and fat.

**Patentansprüche**

1. Verfahren zum Herstellen einer emulgierten Öl-in-Wasser-Nahrungsmittelzusammensetzung, wobei das Verfahren umfasst:

Zugeben von Öl und Fett, enthaltend einen hoch hydrophilen Emulgator, oder einer Mischung von Öl und Fett, enthaltend einen hoch hydrophilen Emulgator und Wasser, zu Wasser oder einer wässrigen Lösung löslicher Mineralien und Vermischen; dann
Zugeben eines Proteins, eines verdickenden Ballaststoffs und optional einer zusätzlichen Komponente; und Erhitzen und Sterilisieren der Zusammensetzung;
wobei
das Öl und Fett eine mittelkettige Fettsäure in einem Anteil von mehr als 0,1 Gew.-% und weniger als 100 Gew.-% bezogen auf die gesamte Fettsäurezusammensetzung enthält,
der hoch hydrophile Emulgator einen HLB-Wert von 6,0 oder mehr aufweist,
der verdickende Ballaststoff ausgewählt ist aus Gellangummi, Carrageen, Pektin, Agar, Xanthangummi, Johannisbrotkernmehl, Alginsäure, Alginaten, Gummi arabicum, Kollagen, Gelatine, Curdlan und Polygamma-Glutaminsäure; und

der pH-Wert der emulgierten Öl-in-Wasser-Nahrungsmittelzusammensetzung unmittelbar nach Herstellung mehr als 5,5 beträgt.

2. Verfahren zum Herstellen einer emulgierten Öl-in-Wasser-Nahrungsmittelzusammensetzung gemäß Anspruch 1, wobei der hochhydrophile Emulgator einen HLB-Wert von 8,8 oder mehr aufweist.

3. Verfahren zum Herstellen einer emulgierten Öl-in-Wasser-Nahrungsmittelzusammensetzung gemäß Anspruch 1 oder 2, wobei der hochhydrophile Emulgator einen HLB-Wert von 11,0 oder mehr aufweist.

4. Verfahren zum Herstellen einer emulgierten Öl-in-Wasser-Nahrungsmittelzusammensetzung gemäß einem der Ansprüche 1 bis 3, wobei das öl und Fett die mittelkettige Fettsäure in einem Gehalt von 7,5 Gew.-% oder mehr und 74,9 Gew.-% oder weniger, bezogen auf die gesamte Fettsäurezusammensetzung, enthält.

5. Verfahren zum Herstellen einer emulgierten Öl-in-Wasser-Nahrungsmittelzusammensetzung gemäß einem der Ansprüche 1 bis 4, wobei die Mischung von Öl und Fett, die einen hoch hydrophilen Emulgator und Wasser enthält, eine vorläufig emulgierte Mischung ist.

6. Verfahren zum Herstellen einer emulgierten Öl-in-Wasser-Nahrungsmittelzusammensetzung gemäß einem der Ansprüche 1 bis 5, wobei das Verfahren Zugeben des verdickenden Ballaststoffs und dann Zugeben des Proteins umfasst.

7. Verfahren zum Herstellen einer emulgierten Öl-in-Wasser-Nahrungsmittelzusammensetzung gemäß einem der Ansprüche 1 bis 5, wobei das Verfahren Zugeben des Proteins und dann Zugeben des verdickenden Ballaststoffs umfasst.

8. Verfahren zum Herstellen einer emulgierten Öl-in-Wasser-Nahrungsmittelzusammensetzung gemäß einem der Ansprüche 1 bis 5, wobei das Verfahren gleichzeitig das Zugeben des Proteins und des verdickenden Ballaststoffs umfasst.

9. Verfahren zum Herstellen einer emulgierten Öl-in-Wasser-Nahrungsmittelzusammensetzung gemäß einem der Ansprüche 1 bis 8, wobei die emulgierte Öl-in-Wasser-Nahrungsmittelzusammensetzung eine Viskosität von 25 cP oder mehr und weniger als 1.000 cP nach dem Erhitzen und Sterilisieren der Zusammensetzung aufweist.

10. Verfahren zum Herstellen einer emulgierten Öl-in-Wasser-Nahrungsmittelzusammensetzung gemäß einem der Ansprüche 1 oder 9, wobei der verdickende Ballaststoff in der emulgierten Öl-in-Wasser-Nahrungsmittelzusammensetzung in einem Gehalt von 0,05 % G/V oder mehr enthalten ist.

11. Verfahren zum Herstellen einer emulgierten Öl-in-Wasser-Nahrungsmittelzusammensetzung gemäß einem der Ansprüche 1 bis 10, wobei der verdickende Ballaststoff Alginsäure und/oder ein Alginat ist.

12. Verfahren zum Herstellen einer emulgierten Öl-in-Wasser-Nahrungsmittelzusammensetzung gemäß einem der Ansprüche 1 bis 11, wobei das Protein in der emulgierten Öl-in-Wasser-Nahrungsmittelzusammensetzung in einem Gehalt von 1,0 % G/V oder mehr, ausgedrückt als Feststoffgehalt, enthalten ist.

13. Emulgierte Öl-in-Wasser-Nahrungsmittelzusammensetzung, erhalten durch das Verfahren zum Herstellen einer emulgierten Öl-in-Wasser-Nahrungsmittelzusammensetzung gemäß einem der Ansprüche 1 bis 12, wobei die Gesamtkonzentration an Emulgatoren in der emulgierten Öl-in-Wasser-Nahrungsmittelzusammensetzung 5,0 Gew.-% oder mehr, bezogen auf Öl und Fett, beträgt.

**Revendications**

1. Procédé de production d'une composition de produit alimentaire émulsionnée huile dans eau, le procédé comprenant :

l'ajout d'une huile et d'une graisse contenant un émulsifiant hautement hydrophile ou un mélange d'une huile et d'une graisse contenant un émulsifiant hautement hydrophile et d'eau à de l'eau ou une solution aqueuse de minéraux solubles dans l'eau et le mélange de ceux-ci ; ensuite

l'ajout d'une protéine, d'une fibre alimentaire épaississante et facultativement d'un composant supplémentaire ; et

le chauffage et la stérilisation de la composition ;

dans lequel

l'huile et la graisse contenant un acide gras à chaîne moyenne dans un rapport de plus de 0,1 % en poids et de moins de 100 % en poids par rapport à la composition totale en acides gras ;

l'émulsifiant hautement hydrophile présente une valeur HLB égale à 6,0 ou plus ;

la fibre alimentaire épaississante est sélectionnée parmi la gomme gellane, la carraghénine, la pectine, l'agar, la gomme xanthane, la gomme de caroube, l'acide alginique, les alginates, la gomme arabique, le collagène, la gélatine, le curdlan et l'acide poly-gamma-glutamique ; et

le pH de la composition de produit alimentaire émulsionnée huile dans eau, immédiatement après la fabrication, est supérieur à 5,5.

2. Procédé de production d'une composition de produit alimentaire émulsionnée huile dans eau selon la revendication 1, dans lequel l'émulsifiant hautement hydrophile présente une valeur HLB égale ou supérieure à 8,0.

3. Procédé de production d'une composition de produit alimentaire émulsionnée huile dans eau selon la revendication 1 ou 2, dans lequel l'émulsifiant hautement hydrophile présente une valeur HLB égale ou supérieure à 11,0.

4. Procédé de production d'une composition de produit alimentaire émulsionnée huile dans eau selon l'une quelconque des revendications 1 à 3, dans lequel l'huile et la graisse contiennent l'acide gras à chaîne moyenne dans un rapport égal ou supérieur à 7,5 % en poids et égal ou inférieur à 74,9 % en poids par rapport à la composition totale en acides gras.

5. Procédé de production d'une composition de produit alimentaire émulsionnée huile dans eau selon l'une quelconque des revendications 1 à 4, dans lequel le mélange d'une huile et d'une graisse contenant un émulsifiant hautement hydrophile et de l'eau est un mélange émulsionné préalablement.

6. Procédé de production d'une composition de produit alimentaire émulsionnée huile dans eau selon l'une quelconque des revendications 1 à 5, le procédé comprenant l'ajout de la fibre alimentaire épaississante et ensuite l'ajout de la protéine.

7. Procédé de production d'une composition de produit alimentaire émulsionnée huile dans eau selon l'une quelconque des revendications 1 à 5, le procédé comprenant l'ajout de la protéine et ensuite l'ajout de la fibre alimentaire épaississante.

8. Procédé de production d'une composition de produit alimentaire émulsionnée huile dans eau selon l'une quelconque des revendications 1 à 5, le procédé comprenant l'ajout de la protéine et de la fibre alimentaire épaississante en même temps.

9. Procédé de production d'une composition de produit alimentaire émulsionnée huile dans eau selon l'une quelconque des revendications 1 à 8, dans lequel la composition de produit alimentaire émulsionnée huile dans eau présente une viscosité égale ou supérieure à 25 cP et inférieure à 1 000 cP après le chauffage et la stérilisation de la composition.

10. Procédé de production d'une composition de produit alimentaire émulsionnée huile dans eau selon l'une quelconque des revendications 1 ou 9, dans lequel la fibre alimentaire épaississante est contenue dans la composition de produit alimentaire émulsionnée huile dans eau en une quantité égale ou supérieure à 0,05 % p/v.

11. Procédé de production d'une composition de produit alimentaire émulsionnée huile dans eau selon l'une quelconque des revendications 1 à 10, dans lequel la fibre alimentaire épaississante est l'acide alginique et/ou un alginate.

12. Procédé de production d'une composition de produit alimentaire émulsionnée huile dans eau selon l'une quelconque des revendications 1 à 11, dans lequel la protéine est contenue dans la composition de produit alimentaire émulsionnée huile dans eau en une quantité égale ou supérieure à 1,0 % p/v en termes de teneur en solides.

13. Composition de produit alimentaire émulsionnée huile dans eau obtenue par le procédé de production d'une composition de produit alimentaire émulsionnée huile dans eau selon l'une quelconque des revendications 1 à12, dans laquelle la concentration totale en émulsifiants dans la composition de produit alimentaire émulsionnée huile dans eau est égale ou supérieure à 5,0 % en poids par rapport à une huile et une graisse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010083774 A
- JP 4047363 B
- WO 2007026474 A
- JP 2009011229 A
- JP 2009106187 A
- EP 2457447 A1
- US 6475539 B1
- US 2008131576 A1
- US 4524086 A
- US 3528823 A
- JP 60025934 B
- JP 2011147444 A

**Non-patent literature cited in the description**

- *Dietary Reference Intakes for Japanese (2010),* 29 May 2009